# EUROPÄISCHE PATENTANMELDUNG

(11) **EP 2 433 516 A1**
(43) Veröffentlichungstag der Anmeldung: **28.03.2012**
(21) Anmeldenummer: 10178857.8
(22) Anmeldetag: 23.09.2010
(51) Int. Cl.: A43D 1/02, A61B 5/00, A61B 5/103

(54) **Servicesystem zum Bestimmen eines nutzerspezifischen Schuhartikels sowie Verfahren**

(71) Anmelder: Schelchen GmbH, 15711 Zeesen (DE)
(72) Erfinder: Bürger, Tilo, Dipl.-Ing., 15754 Heidesee, OT Wolzig (DE); Timm, Thomas, 12309 Berlin (DE)
(74) Vertreter: Bittner, Thomas L.

(57) **Zusammenfassung**

Die Erfindung betrifft ein Servicesystem zum Bestimmen eines nutzerspezifischen Schuhartikels, mit einer Messeinrichtung (1) mit einem mehrfach verwendbaren Thermoschichtmaterial (2), auf dem eine Auftrittsfläche (3) zum Aufsetzen eines Fußes eines Nutzers für eine Fußabdruckerfassung gebildet ist, einer Kamera- oder Scanneinrichtung (4), die konfiguriert ist, digitale Bilddaten für einen Fußabdruck im Bereich der Auftrittsfläche (3) nach dem Aufsetzen und Entfernen des Fußes des Nutzers aufzunehmen, einer Auswerteeinrichtung (5), die konfiguriert ist, wenigstens einen Fußeigenschaftsparameter für den Fuß des Nutzers zu bestimmen, einer Speichereinrichtung (7) mit elektronischen Produktinformationen, die eine Zuordnung zwischen Fußeigenschaftsparametern und Schuhartikeln enthalten, einer Empfehlungseinrichtung (6), die konfiguriert ist, den wenigstens einen Fußeigenschaftsparameter für den Fuß des Nutzers und die elektronischen Produktinformationen vergleichend auszuwerten und hieraus eine elektronische Empfehlungsinformation für einen der Schuhartikel zu erzeugen, und eine Ausgabeeinrichtung (8), die konfiguriert ist, die elektronische Empfehlungsinformation auszugeben. Des Weiteren betrifft die Erfindung ein Verfahren zum Bestimmen eines nutzerspezifischen Schuhartikels. Fig.

## Beschreibung

Die Erfindung betrifft ein Servicesystem zum Bestimmen eines nutzerspezifischen Schuhartikels sowie ein Verfahren.

### Hintergrund der Erfindung

Es wurden verschiedentlich Vorrichtungen und Verfahren zum Bestimmen von Eigenschaften der Füße von Personen vorgeschlagen. Beispielsweise beschreibt das Dokument US 4,534,365 eine Vorrichtung zum Evaluieren von Fußeigenschaften einer Person hinsichtlich der Blutzirkulation und der anatomischen Form. Hierzu wird eine Vorrichtung vorgeschlagen, bei der der Nutzer mit seinen Füßen auf einer ebenen transparenten Fläche steht, die mit einem thermografischen Film versehen ist. Spiegel unterhalb der Standfläche werden genutzt, um von unten auf die Fußflächen zu schauen.

Das Dokument DE 44 16 731 A1 offenbart ein Verfahren zum Ermitteln der Kontur und der Auftrittsfläche von Füßen. Hierbei ist vorgesehen, dass der Fuß auf einem Flüssigkristalle aufweisenden Folienabschnitt aufgedrückt, in Abhängigkeit der Fußtemperatur durch die Flüssigkeitskristalle über einen Zeitabschnitt ein Reflexionsfarbbild erzeugt und nachfolgend das Reflexionsfarbbild als Auftrittsfläche des Fußes abgegriffen wird. Der Abgriff des Reflexionsfarbbildes kann mechanisch oder mittels fotografischer, elektronischer oder physikalischer Medien erfolgen, beispielsweise einem Scanner. Das Reflexionsfarbbild wird benutzt, um spezifische Fußdaten zu ermitteln für die verbesserte Herstellung oder Anpassung von Schuhwerk.

Im Dokument US 5,790,256 sind eine Vorrichtung und ein Verfahren zum Analysieren von Füßen beschrieben, wobei eine Anordnung von Drucksensoren und optischen Sensoren, die an eine Steuereinrichtung und einem Monitor angeschlossen sind, verwendet wird. Die optischen Sensoren, die um die Füße herum angeordnet sind, dienen zum Messen der Länge, der Breite und der Höhe des Fußes. Mit Hilfe der Drucksensoren wird ein Fußabdruck bestimmt. Im Dokument WO 2008/073430 A2 ist eine Messeinrichtung zur Temperaturmessung an einer Fußsohle offenbart. Schließlich betrifft das Dokument EP 1 844 709 A1 ein Verfahren und eine Anordnung zum Bestimmen einer Innensohlenstruktur, die an den Fuß eines Nutzers angepasst ist. Hierbei ist vorgesehen, einen Fuß oder beide Füße des Nutzers auf einem thermosensitiven Schichtmaterial anzuordnen und den Fußabdruck anschließend zu analysieren, um einen Fußtyp einer Kategorisierung entsprechend zu bestimmen. Zusätzlich ist vorgesehen, die statische Beinachse des Nutzers zu ermitteln.

Die bekannten Verfahren und Vorrichtungen lösen Einzelaspekte in Verbindung mit dem Bestimmen von Eigenschaften von Füßen. Auch ist teilweise vorgesehen, ermittelte Fußeigenschaftsparameter bei der Herstellung von Schuhwerk zu berücksichtigen. Es besteht jedoch Bedarf für ein System, welches die Bestimmung und Berücksichtigung von Fußeigenschaften in einer üblichen Verkaufsumgebung ermöglicht, insbesondere im Einzelhandel.

### Zusammenfassung der Erfindung

Aufgabe der Erfindung ist es, ein Servicesystem zum Bestimmen eines nutzerspezifischen Schuhartikels zu schaffen, welches für den massenhaften täglichen Einsatz geeignet ist, insbesondere im Einzelhandel.

Diese Aufgabe wird erfindungsgemäß durch eine Servicesystem zum Bestimmen eines nutzerspezifischen Schuhartikels, insbesondere eines Schuheinlageartikels nach den unabhängigen Anspruch 1 sowie ein Verfahren nach dem unabhängigen Anspruch 8 gelöst. Vorteilhafte Ausgestaltungen der Erfindung sind Gegenstand von abhängigen Unteransprüchen.

Die Erfindung umfasst den Gedanken eines Servicesystems zum Bestimmen eines nutzerspezifischen Schuhartikels, insbesondere eines Schuheinlageartikels, welches zunächst eine Messeinrichtung mit einem mehrfach verwendbaren Thermoschichtmaterial umfasst, auf dem eine Auftrittsfläche zum Aufsetzen eines Fußes eines Nutzers für eine Fußabdruckerfassung gebildet ist. Der für den Nutzer charakteristische Fußabdruck entsteht beim Aufsetzen des Fußes auf die Auftrittsfläche dadurch, dass sich das Thermoschichtmaterial in Abhängigkeit von der Temperatur der Fußsohle verändert, insbesondere indem es sich verfärbt. Diese Veränderung findet insbesondere in den Bereichen statt, die mit der Fußsohle direkt in Kontakt kommen. Demgegenüber wird die Veränderung des Thermoschichtmaterials beispielsweise im Bereich des Fußgewölbes kaum stattfinden. Es entsteht so ein für den Nutzer spezifischer Fußabdruck.

Das Servicesystem weist eine Kamera- oder Scaneinrichtung auf, die konfiguriert ist, digitale Bilddaten für einen Fußabdruck im Bereich der Auftrittsfläche nach dem Aufsetzen und dem Entfernen des Fußes des Nutzers aufzunehmen. Hierbei kann es sich um eine beliebige digitale Kameraeinrichtung handeln, wie sie heute in verschiedensten Ausgestaltungen als solche verfügbar sind. Das mit Hilfe der Kamera- oder Scaneinrichtung erzeugte Bild kann ein farbiges Bild oder ein Schwarz-Weiß-Bild sein. Die auf diese Weise erzeugten digitalen Bilddaten werden anschließend mit Hilfe einer von dem Servicesystem umfassten Auswerteeinrichtung ausgewertet, die an die Kamera- oder Scaneinrichtung datentechnisch gekoppelt und konfiguriert ist, mit Hilfe der Bilddatenauswertung wenigstens einen Fußeigenschaftsparameter für den Fuß des Nutzers zu bestimmen. Es kann auch vorgesehen sein, dass mehrere Fußeigenschaftsparameter bei der Bilddatenauswertung bestimmt werden. In einer Ausgestaltung kann vorgesehen sein, anhand der Bilddatenauswertung einen von mehreren Fußtypen zu bestimmen. So ist es beispielsweise bekannt, dass bei einem Hohlfuß ein überhöhtes Fußgewölbe gebildet ist, was anhand des Fußabdrucks erkennbar ist. Es treten in diesem Fall besondere Belastungszonen im Bereich hinter der kleinen Zehe sowie im Hackenbereich unterhalb des Fersenbeins auf. Ähnlich können andere Fußtypen bestimmt werden.

Das Servicesystem verfügt weiterhin über eine Speichereinrichtung, in der elektronische Produkt- oder Artikelinformationen gespeichert sind, die eine Zuordnung zwischen Fußeigenschaftsparametern und Schuhartikeln enthalten. Dieses bedeutet beispielsweise, dass die von einer oder mehreren Firmen angebotenen Schuhartikel in Kategorien eingeteilt sind, denen jeweils ein oder mehrere charakteristische Fußeigenschaftsparameter zugeordnet werden. Die elektronische Produktinformation sowie das Ergebnis der Bilddatenanalyse in Form des wenigstens einen Fußeigenschaftsparameter können dann von einer von dem Servicesystem umfassten Empfehlungseinrichtung ausgewertet werden, derart, dass hieraus eine elektronische Empfehlungsinformation für einen oder mehrere der Schuhartikel erzeugt wird. Die Empfehlungseinrichtung ist wenigstens zeitweise an die Auswerteeinrichtung und die Speichereinrichtung datentechnisch gekoppelt, um den wenigstens einen Fußeigenschaftsparameter aus der Bilddatenanalyse sowie die elektronische Produktinformation zumindest teilweise zu Empfangen.

Das Servicesystem umfasst schließlich eine Ausgabeeinrichtung, die an die Empfehlungseinrichtung datentechnisch gekoppelt ist. Die Ausgabeeinrichtung ist konfiguriert, die elektronische Empfehlungsinformation von der Empfehlungseinrichtung zu empfangen und auszugeben. Die Ausgabe kann beispielsweise über eine Anzeige oder eine Druckereinrichtung erfolgen.

Die Datenkommunikation zwischen den einzelnen Einrichtungen des Servicesystems kann drahtlose und / oder kabelgebundene Datenverbindungen umfassen, die dauerhaft bestehen oder im Bedarfsfall etabliert werden.

Mit Hilfe des vorgeschlagenen Servicesystems sowie des Verfahrens zum Bestimmen eines nutzerspezifischen Schuhartikels sind Technologien geschaffen, die auch bei einer Massennutzung das Bestimmen von Fußeigenschaften und das automatische Zuordnen eines oder mehrerer geeigneter Schuhartikel zuverlässig gewährleisten. Auch beim täglichen Einsatz im Schuheinzelhandel kann der potentielle Käufer zielgerichtet mit geeigneten Schuhartikeln versorgt werden, wahlweise sogar ohne jegliche ergänzende Beratung durch das Verkaufspersonal.

Eine bevorzugte Weiterbildung der Erfindung sieht vor, dass mehrere Systemkomponenten, nämlich die Kamera- oder Scanneinrichtung, die Auswerteeinrichtung, die Speichereinrichtung, die Empfehlungseinrichtung und / oder die Ausgabeeinrichtung, in ein einziges Datenverarbeitungssystem integriert sind. Ein solches Datenverarbeitungssystem kann beispielsweise ein hardware- und softwaretechnisch entsprechend eingerichtetes Computersystem sein. Einzelne Einrichtungen, beispielsweise die Auswerteeinrichtung, sind bei dieser oder anderen Ausführungsformen zweckmäßigerweise mit Hilfe eines entsprechenden Softwaremoduls implementiert.

Bei einer zweckmäßigen Ausgestaltung der Erfindung kann vorgesehen sein, dass zumindest die Kamera- oder Scanneinrichtung in ein mobiles Gerät integriert ist. Hierbei handelt es sich um eine bevorzugte Ausgestaltung im Unterscheid zur ebenfalls möglichen Ausführung, dass zumindest die Kamera- oder Scanneinrichtung in ein stationäres Gerät integriert ist. Beispielsweise kann es sich bei dem mobilen Gerät um ein Mobilfunktelefon mit einer digitalen Kamera handeln. Auch kann vorgesehen sein, die digitalen Bilddaten für den Fußabdruck mit Hilfe einer so genannten Web-Kamera aufzunehmen, die an ein Netzwerk angeschlossen ist, insbesondere das Internet. In Verbindung mit der Nutzung eines Mobiltelefons kann vorgesehen sein, dass auf diesem eine Software-Applikation implementiert ist, die anschließend die Auswertung der Bilddaten durchführt und mittels Zugriff auf die elektronischen Produktinformationen, bevorzugt über eine drahtlose Datenverbindung auf eine zentrale Servereinrichtung, die Empfehlungsinformation erzeugt und über die Anzeige des Mobiltelefons ausgibt. Auf diese Weise ist dem Nutzer eine individuelle Nutzung des Servicesystems mit Hilfe seines eigenen Mobilfunktelefons ermöglicht.

Eine vorteilhafte Ausführungsform der Erfindung sieht vor, dass zumindest die Auswerteeinrichtung unter Nutzung einer lokalen Softwareapplikation in dem mobilen Gerät implementiert ist.

Bevorzugt sieht eine Fortbildung der Erfindung vor, dass zumindest die Auswerteeinrichtung in einer zentralen Servereinrichtung gebildet ist. In der zentralen Servereinrichtung können weitere Systemkomponenten implementiert sein, beispielsweise die Speichereinrichtung und / oder die Empfehlungseinrichtung. Eine Ausgestaltung sieht vor, dass das System mit Hilfe der Client-Server-Technologie implementiert ist.

Bei einer vorteilhaften Ausgestaltung der Erfindung kann vorgesehen sein, dass das mehrfach verwendbare Thermoschichtmaterial in der Messeinrichtung auf einer rutschfesten Unterlage angeordnet ist.

Eine Weiterbildung der Erfindung kann vorsehen, dass das mehrfach verwendbare Thermoschichtmaterial austauschbar in der Messeinrichtung angeordnet ist.

In Verbindung mit dem Verfahren zum Bestimmen eines nutzerspezifischen Schuhartikels, insbesondere eines Schuheinlageartikels, können die im Zusammenhang mit dem Servicesystem beschriebenen vorteilhaften Ausgestaltungen entsprechend einzeln oder in beliebiger Kombination vorgesehen sein. So kann beispielsweise vorgesehen sein, das Bestimmen des wenigstens einen Fußeigenschaftsparameters mittels Bilddatenanalyse und / oder das Erzeugen der elektronischen Empfehlungsinformation in einer zentralen Servereinrichtung auszuführen. Alternativ kann dieses in einem mobilen Gerät, beispielsweise einem Mobilfunktelefon, ausgeführt werden.

### Beschreibung bevorzugter Ausführungsbeispiele der Erfindung

Die Erfindung wird im Folgenden anhand von bevorzugten Ausführungsbeispielen unter Bezugnahme auf eine Figur näher erläutert.

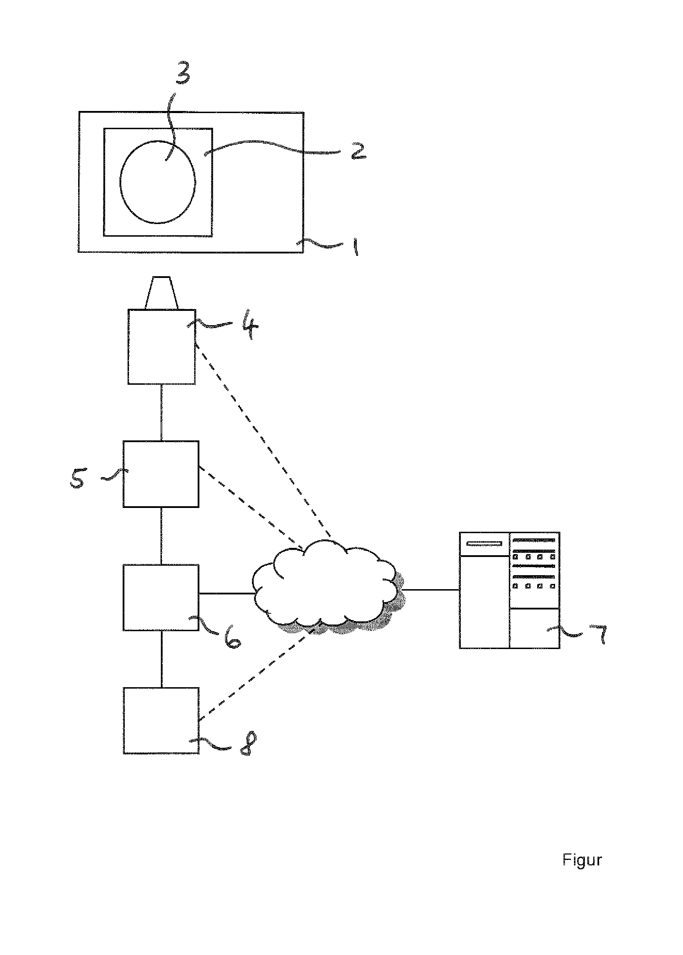

Die einzige Figur zeigt eine schematische Darstellung eines Servicesystems zum Bestimmen eines nutzerspezifischen Schuhartikels, insbesondere eines Schuheinlageartikels.

Eine Messeinrichtung 1 ist mit einem mehrfach verwendbaren Thermoschichtmaterial 2 gebildet, auf dem eine Auftrittsfläche 3 zum Aufsetzen eines Fußes oder beider Füße eines Nutzers gebildet ist, um auf diese Weise unter Ausnutzung der Veränderung des Thermoschichtmaterials 2 aufgrund des Aufsetzen des Fußes einen Fußabdruck zu erfassen.

Der Fußabdruck wird mit Hilfe einer Kamera 4 eingescannt, so dass digitale Bilddaten für den Fußabdruck im Bereich der Auftrittsfläche 3 erzeugt werden. Eine an die Kamera 4 angeschlossene Auswerteeinrichtung 5 empfängt die digitalen Bilddaten und wertet diese aus, um hieraus einen oder mehrere Fußeigenschaftsparameter für den Fuß des Nutzers zu bestimmen. Die Bilddatenanalyse kann auf verschiedene Art und Weise erfolgen. Es sind Bildauswerteverfahren als solche in verschiedenen Ausgestaltungen bekannt, weshalb dieses hier nicht weiter erläutert wird. Beispielsweise kann die Bestimmung des einen oder der mehreren Fußeigenschaftsparameter genutzt werden, um einen Fußtyp für den Nutzer zu bestimmen. Beispiel für einen Fußtyp ist der sogenannte Hohlfuß, welcher sich insbesondere durch eine verringerte Abdruckfläche im Vergleich zum Normalfuß auszeichnet.

Der eine oder die mehreren Fußeigenschaftsparameter werden dann in einer Empfehlungseinrichtung 6 bereitgestellt, um dort aus einem Vergleich der Fußeigenschaftsparameter mit elektronischen Produktinformationen aus einer Speichereinrichtung 7 eine elektronische Empfehlungsinformation zu erzeugen. Im dargestellten Ausführungsbeispiel ist die Speichereinrichtung 7 in einer zentralen Servereinrichtung implementiert, auf die über einer Datenleitung zugegriffen werden kann. In anderen Ausführungen haben auch andere Systemkomponenten Zugriff auf die zentrale Servereinrichtung, was in der Figur mittels gestrichelter Linien gezeigt ist. Auch kann vorgesehen sein, dass ergänzend zu der Speichereinrichtung 7 weitere oder andere Systemkomponenten in der zentralen Servereinrichtung implementiert sind.

Die elektronische Produktinformation unterteilt in einem Ausführungsbeispiel die Schuhartikel eines oder mehrerer Unternehmen in eine Vielzahl von Kategorien, die ihrerseits jeweils durch einen Fußeigenschaftsparameter oder eine Kombination von mehreren Fußeigenschaftsparametern bestimmt sind. Aus dem Vergleich mit dem einen oder dem mehreren aus der Bilddatenanalyse ermittelten Fußeigenschaftsparametern ermittelt die Empfehlungseinrichtung dann eine oder mehrere Schuhartikel aus den Kategorien, die den bestimmten Fußeigenschaftsparametern entsprechen.

Die von der Empfehlungseinrichtung erzeugte elektronische Empfehlungsinformation wird dann auf eine Ausgabeeinrichtung 8 gegeben, die eine Ausgabe erzeugt, beispielsweise auf einer Anzeige und / oder über eine Druckereinrichtung.

In einer Ausführung ist vorgesehen, dass die Auswerteeinrichtung 5 in einem zentralen Server gebildet ist, auf den von verschiedenen Verkaufsbereichen aus zugegriffen werden kann. Aber auch eine lokale Auswertung vor Ort in einer entsprechend ausgerüsteten Datenverarbeitungsanlage kann vorgesehen sein. Der Zugriff auf die Auswerteeinrichtung 5 in dem zentralen Server kann über eine kabellose oder eine drahtgebundene Datenverbindung erfolgen. Alternativ oder ergänzend kann die Speichereinrichtung mit den elektronischen Produktinformationen in dem zentralen Server oder einem anderen Server gebildet sein.

Die in der vorstehenden Beschreibung, den Ansprüchen und der Figur offenbarten Merkmale der Erfindung können sowohl einzeln als auch in beliebiger Kombination für die Verwirklichung der Erfindung in ihren verschiedenen Ausführungen von Bedeutung sein.

## Patentansprüche

1. Servicesystem zum Bestimmen eines nutzerspezifischen Schuhartikels, insbesondere eines Schuheinlageartikels, mit:
- einer Messeinrichtung (1) mit einem mehrfach verwendbaren Thermoschichtmaterial (2), auf dem eine Auftrittsfläche (3) zum Aufsetzen eines Fußes eines Nutzers für eine Fußabdruckerfassung gebildet ist,
- einer Kamera- oder Scanneinrichtung (4), die konfiguriert ist, digitale Bilddaten für einen Fußabdruck im Bereich der Auftrittsfläche (3) nach dem Aufsetzen und Entfernen des Fußes des Nutzers aufzunehmen,
- einer Auswerteeinrichtung (5), die an die Kamera- oder Scanneinrichtung (4) datentechnisch gekoppelt und konfiguriert ist, wenigstens einen Fußeigenschaftsparameter für den Fuß des Nutzers zu bestimmen, indem zumindest die digitalen Bilddaten ausgewertet werden,
- einer Speichereinrichtung (7) mit elektronischen Produktinformationen, die eine Zuordnung zwischen Fußeigenschaftsparametern und Schuhartikeln enthalten,
- einer Empfehlungseinrichtung (6), die an die Auswerteeinrichtung (5) und die Speichereinrichtung (7) datentechnisch gekoppelt und konfiguriert ist, den wenigstens einen Fußeigenschaftsparameter für den Fuß des Nutzers und die elektronischen Produktinformationen vergleichend auszuwerten und hieraus eine elektronische Empfehlungsinformation für einen der Schuhartikel zu erzeugen, und
- eine Ausgabeeinrichtung (8), die an die Empfehlungseinrichtung (6) datentechnisch gekoppelt und konfiguriert ist, die elektronische Empfehlungsinformation auszugeben.

2. Servicesystem nach Anspruch 1, **dadurch gekennzeichnet, dass** mehrere Systemkomponenten, nämlich die Kamera- oder Scanneinrichtung (4), die Auswerteeinrichtung (5), die Speichereinrichtung (7), die Empfehlungseinrichtung (6) und / oder die Ausgabeeinrichtung (8), in ein einziges Datenverarbeitungssystem integriert sind.

3. Servicesystem nach Anspruch 1 oder 2, **dadurch gekennzeichnet, dass** zumindest die Kamera- oder Scanneinrichtung (4) in ein mobiles Gerät integriert ist.

4. Servicesystem nach Anspruch 3, **dadurch gekennzeichnet, dass** zumindest die Auswerteeinrichtung (5) unter Nutzung einer lokalen Softwareapplikation in dem mobilen Gerät implementiert ist.

5. Servicesystem nach mindestens einem der Ansprüche 1 bis 3, **dadurch gekennzeichnet, dass** zumindest die Auswerteeinrichtung (5) in einer zentralen Servereinrichtung gebildet ist.

6. Servicesystem nach mindestens einem der vorangehenden Ansprüche, **dadurch gekennzeichnet, dass** das mehrfach verwendbare Thermoschichtmaterial in der Messeinrichtung auf einer rutschfesten Unterlage angeordnet ist.

7. Servicesystem nach mindestens einem der vorangehenden Ansprüche, **dadurch gekennzeichnet, dass** das mehrfach verwendbare Thermoschichtmaterial (2) austauschbar in der Messeinrichtung (1) angeordnet ist.

8. Verfahren zum Bestimmen eines nutzerspezifischen Schuhartikels, insbesondere eines Schuheinlageartikels, in einem Servicesystem, wobei das Verfahren die folgenden Schritte umfasst:
- Erfassen eines Fußabdrucks in einer Messeinrichtung mittels eines mehrfach verwendbaren Thermoschichtmaterials, auf dem eine Auftrittsfläche zum Aufsetzen eines Fußes eines Nutzer gebildet ist,
- Aufnehmen digitaler Bilddaten für den Fußabdruck im Bereich der Auftrittsfläche nach dem Aufsetzen und Entfernen des Fußes des Nutzers mittels einer Kamera- oder Scanneinrichtung,
- Bestimmen wenigstens einen Fußeigenschaftsparameter für den Fuß des Nutzers, indem zumindest die digitalen Bilddaten mittels einer Auswerteeinrichtung ausgewertet werden, die an die Kamera- oder Scanneinrichtung datentechnisch gekoppelt ist,
- Bereitstellen von elektronischen Produktinformationen, die eine Zuordnung zwischen Fußeigenschaftsparametern und Schuhartikeln enthalten, in einer Speichereinrichtung,
- vergleichendes Auswerten des wenigstens einen Fußeigenschaftsparameters für den Fuß des Nutzers und der elektronischen Produktinformationen und Erzeugen einer elektronische Empfehlungsinformation für einen der Schuhartikel in einer Empfehlungseinrichtung, die an die Auswerteeinrichtung und die Speichereinrichtung datentechnisch gekoppelt ist, und
- Ausgeben elektronischen Empfehlungsinformation über eine Ausgabeeinrichtung, die an die Empfehlungseinrichtung datentechnisch gekoppelt ist.
